# EUROPEAN PATENT SPECIFICATION

(11) **EP 1 181 349 B1**
(45) Date of publication and mention of the grant of the patent: **01.03.2006**
(21) Application number: 00935356.6
(22) Date of filing: 30.05.2000
(51) Int. Cl.: C12M 3/06

(54) **CULTURE CHAMBER**
VORRICHTUNG ZUR ZÜCHTUNG VON ZELLKULTUREN
ENCEINTE DE CULTURE

(30) Priority: 28.05.1999 GB 9912641; 28.10.1999 WO PCT/GB99/03558
(43) Date of publication of application: 27.02.2002
(73) Proprietor: Cellon S.A., 7213 Bereldange (LU)
(72) Inventor: FULLER, Jess Paul, Leicestershire LE67 2FP (GB); CLAYSON, Tony, Leicestershire LE67 2LR (GB); BIRD, Robert McLean, Derbyshire DE17 3FR (GB); CLIFFORD, Timothy Burgess, Leicestershire LE7 3FE (GB); KNIGHTS, Anthony James, Bishopston, Swansea SA3 3JY (GB)
(74) Representative: Gill, Siân Victoria
(86) International application number: PCT/GB2000/002073
(87) International publication number: WO 2001/092462

(56) References cited:
- WO-A-97/08291
- WO-A-99/55827
- US-A- 4 939 151
- US-A- 5 686 304
- US-A- 5 702 945
- US-A- 5 786 215

## Description

The present invention relates to culture chambers for use in a method of culturing microbiological material and, in particular, to culture chambers that provide increased gaseous exchange between the interior and exterior of the culture chamber and increased surface area for cell adhesion.

The general principles of culturing cells *in vitro* are well-established in the field of biotechnology, with the term "cell culture" being generally understood to refer to the growth and maintenance of cells, either as a cell suspension or an attached monolayer, usually devoid of structural organisation. "Tissue culture" has become the generic term encompassing culture of small-fragments-of-animal, insect or plant tissue or whole embryonic organs, explanted to retain tissue architecture.

The cells in tissue and cell culture require oxygen and nutrients in order to grow and be maintained. There must also be provision for the removal of carbon dioxide and other metabolic waste products. The cells are generally provided with nutrients by way of the medium in which the culturing takes place. Different cells have different requirements and suitable nutrients may be included in the medium in order to meet the specific requirements of the type of cells being cultured. The culture chamber may be arranged to allow a throughput of nutrient medium, thereby providing a constant source of nutrients and removing waste products which are released into the medium by the cells. Providing the necessary gas exchange, that is the provision of oxygen and the removal of gaseous waste products such as CO₂, can be more problematic and often represents a serious limiting factor on cell and tissue culture. Many different ways of providing cells in culture with oxygen have been used, with varying degrees of success. One method is agitation of the culture chamber so that air is mixed in or the cells are exposed to the surface and therefore to oxygen. This method of oxygenation is most effective when the culture chamber contains as small a volume of medium compared to the chamber surface area as possible. Thus, the volume of nutrient medium one can use is severely limited when oxygen is supplied to the cells in this manner. An alternative method involves bubbling of oxygen-containing gases through the culture medium. However, whilst this method successfully provides the cells with oxygen, the bubbles can damage the relatively delicate cells.

The favoured method of providing cells in culture with oxygen is provided by diffusion into the culture chamber through a gas-permeable membrane. This diffusion overcomes the above mentioned problems associated with agitation and bubbling. The gas-permeable membrane may be in the form of tubes, or the like, passing through the culture chamber. Oxygen is then passed through said tubes and diffuses through the tube walls into the culture chamber. Alternatively, a wall or part of a wall of the culture chamber itself may be gas-permeable and thus allow oxygen to diffuse through said wall or wall portion and into the culture chamber. The amount of oxygen which is able to diffuse into the culture chamber is limited by the membrane surface area and gas-permeability. It should be noted that the membrane must be non-porous and must have some degree of structural strength, especially if it forms a wall or part of a wall of the culture chamber.

Despite the above described methods for providing gaseous exchange between the culture chamber interior and exterior, it has been found that the rate at which oxygen can be provided and gaseous waste products can be removed places a limit on the growth and proliferation of the cells. As the cells inside the culture chamber grow and proliferate, the oxygen requirement will increase and the waste products will build up, eventually preventing further growth and proliferation.

A further potential limiting factor encountered with known culture chambers is the surface area of the interior of the culture chamber available for cell adherence. There are two major types of cells grown *in vitro*, anchorage-dependent cells which require attachment to a growth substrate and anchorage-independent cells which can multiply *in vitro* without being attached to a surface. A smooth substrate, that is a smooth interior surface of the culture chamber provides a limited surface area for anchorage-dependent cells to attach and so will limit cell growth and proliferation. Furthermore, whilst many cells can attach to a smooth surface, certain "problem" cell types are unable to attach to a smooth surface.

A number of methods of increasing the surface area of the growth substrate in a culture chamber are known. These methods include elaborate structures which are placed within the culture chamber to provide additional cell attachment surfaces. The introduction of microcarriers into culture chambers is also know, the microcarriers comprising, for example, small glass spheres or sodium alginate.

US Patent No. 5,702,945 discloses culture vessels which are said to attain optimal gas exchange, nutrient supply and disposal of metabolic waste products without contamination during high-density cultivation of adherent cells. The vessel comprises a gas-permeable gas exchange membrane, a dialysis membrane and a three-dimensional carrier on which the cells are cultivated.

US Patent No. 5,786,215 discloses a cell culture apparatus including an integral culture and gassing element formed from spirally-wound or concentrically-wrapped lengths of liquid-impermeable, oxygen-permeable membrane envelope.

US Patent No. 5,686,304 discloses a cell culture apparatus formed of thin, spaced, gas-permeable silicone membranes sealed at their edges to form bag-like vessels comprising one or more interior chambers for containing cell culture media. A suitable portion of the membrane surfaces are of a suitable thickness and surface area to provide structural integrity and sufficient gas permeability for cell growth within the chamber.

International Publication No. WO 97/08291 discloses an apparatus for culturing biological material providing a textured internal surface for cell attachment. A method for forming the textured surface is also disclosed.

International Publication No. WO 99/55827 falls under the provisions of Article 54(3) EPC and discloses an apparatus for performing cell growth and cell based assays. The apparatus comprises a plurality of micro-channel elements, each micro-channel element comprising a cell growth chamber, an inlet channel and an outlet channel.

It is an object of the present invention to provide a culture chamber which overcomes the above discussed limitations of the known culture chambers. According to a first aspect of the invention, a culture chamber for use in a method of culturing microbiological material is provided, the culture chamber having a wall or at least a portion of a wall consisting of a gas-permeable membrane, a textured interior growth surface arranged for contact with the microbiological material being cultured and at least a portion of the external surface gas-permeable membrane being textured to enhance gas permeability of the membrane.

The textured interior growth surface is formed on the interior surface of the gas-permeable membrane. Preferably, the textured interior and exterior surfaces of the gas-petmeable membrane overlap with one another, so that part of the gas-permeable membrane is textured on both of its surfaces. In an alternative embodiment, the textured portions on the interior and exterior of the gas-permeable membrane do not overlap. Herein, the term "overlap" means that the textured surfaces are substantially coincident on the opposite sides of the membrane.

A textured surface of the gas-permeable membrane increases the surface area through which gases may permeate. The permeability of the membrane itself will be limited by the area of both of its two surfaces. Thus, if one surface of the membrane is smooth and the other textured, the permeability of the membrane will be limited by the smaller surface area of the smooth surface. Thus, in order to benefit properly from the increased gas-permeability of the membrane afforded by a textured surface, both surfaces of the membrane must be textured. Ideally, at least 25% of the gas-permeable membrane is textured on both sides, more preferably, at least 50% is textured on both sides, and most preferably at least 90% of the gas-permeable membrane is textured on both its interior and exterior surfaces. This means that the degree of overlap of the textured surfaces is preferably at least 25%, more preferably 50%, and most preferably 90%. The textured surface on the culture chamber interior has the added advantage that it provides a greater area for cell attachment. Where both surfaces of the gas-permeable membrane are textured, the oxygen diffusing into the culture chamber is fed directly to the cells attached to the interior surface.

In a preferred embodiment, the gas-permeable wall and the textured surfaces are each formed from an organic polymer, optionally the same organic polymer. As the gas-permeable wall or portion of a wall of the culture chamber also provides the textured interior growth surface, cells may grow directly on a textured growth surface on the gas-permeable membrane, thus allowing high cell densities. The gas-permeable wall also has a textured exterior surface, thereby enhancing gas-permeability of the wall in particular in close proximity to the cells growing on the interior surface of the wall.

In a preferred embodiment, the gas-permeable membrane comprises silicone. Silicones surpass other elastomers in many performance categories because of their rigid silicon-oxygen chemical structure. The process of vulcanisation transforms this structure, allowing the silicon-oxygen polymer to become an elastic rubber. Silicone rubbers are stable throughout a temperature range of -46°C to 232°C. They are odourless and tasteless. Silicone rubbers also do not stain or corrode with other materials. Advantageously, silicone rubbers are also not physically or chemically degraded or altered by contact with body fluids. Silicone rubbers can be formulated and tested for full bio-compatibility and compliance with guidelines for medical products. A further and particularly important advantage of silicone rubbers is that they have relatively high oxygen permeability within the scope of known polymers.

Preferably, at least one of the textured surfaces of the gas-permeable membrane is a layer of textured silicone. In a preferred embodiment of the invention, the textured silicone rubber layer is formed by a method comprising forming a coating of a silicone rubber precursor on a substrate, contacting a surface of the coating with a biologically-acceptable sacrificial filler, curing the resultant mixture and removing the sacrificial filler to form a layer of silicone rubber with a textured surface. In a preferred embodiment, the surface of the coating is contacted with the sacrificial filler under gravity, such that the sacrificial filler is substantially completely embedded in the coating. Alternatively, the sacrificial filler may be sprayed on to the surface of the coating, or may be applied loosely to the surface of the coating and then embedded by contacting the surface with a pressure roller. Preferably, the sacrificial filler is embedded to a depth of 0.1-1.0 mm, more preferably 0.1-0.5 mm, and most preferably 0.1-0.25 mm. In a preferred embodiment, a thicker layer of uncured silicone rubber is used. When the sacrificial filler is then sprinkled onto this thick layer, some particles will sink into the uncured silicone and other particles become positioned on top of them. When the rubber is cured and the sacrificial filler removed, these particles will create deep craters or channels which are particularly good for promoting cell adhesion. This thicker textured silicone layer may be applied to either surface of the gas-permeable membrane, or to both. Preferably, the thicker layer of textured silicone is twice as thick as a normal silicone layer. In an alternative embodiment, the sacrificial filler is scattered or sprinkled over the surface of the coating, such that the sacrificial filler is only partially embedded in the surface. The latter technique can be used to provide the surface of the silicone rubber with a less uniform texture that is particularly suitable for growing certain types of adherent cells. Preferably, the resultant textured surface is micro-cupulated, i.e., cratered or pitted, the micro-cupules having a depth of less than 1 mm, preferably a depth of 0.5-0.1 mm. In a preferred embodiment, the micro-cupules measure less than 2 mm across, preferably less than 1 mm across, and, most preferably, less than 0.5 mm across. Silicone rubbers are available with a wide range of different physical properties, both in the uncured and cured state, and their methods of cure also differ widely. Consequently, the nature and properties of the silicone rubber used can affect the manufacturing process and the choice of a suitable silicone rubber precursor can be important. The silicone rubber precursor should be selected with due consideration to the manner in which the mixture is to be applied to the substrate, the conditions required for curing, and the desired properties of the end product. The uncured silicone rubber should usually have an appropriate viscosity for the method of its application to the substrate, and should retain its general form once the sacrificial filler has adhered to its surface. The conditions for curing must generally be compatible with both the substrate to which the uncured silicone rubber is applied and the sacrificial filler that adheres to the surface. Finally, the quality of the silicone rubber used should be selected in light of the intended application of the final product. In an especially preferred embodiment, silicone rubber paint RTV 118 (General Electric Co., Connecticut, USA) or R2-113 (NuSil Technology, California, USA) are used. Silicone rubber precursors which are widely available commercially may be used, for example, from Dow Chemical Corporation, Midland, Minnesota, USA, or from GE Silicones Europe, Bergen op Zoom, the Netherlands. In a preferred embodiment, the silicone rubber precursor is one that can be cured or vulcanised at room temperature. This obviates the need to expose the mixture to elevated temperatures, which is particularly useful as some sacrificial fillers become unstable and decompose at elevated temperatures, thus making it difficult to control the final form of the structured silicone rubber. In order to assist adhesion of the silicone rubber layer to certain materials, it may be necessary to apply a conventional adhesive, such as a mineral spirit-based primer, prior to deposition of the silicone layer. In a preferred embodiment, the primer used is silicone rubber primer SS 4155 (General Electric Co., Connecticut, USA).

Preferably, the biologically acceptable sacrificial filler used in the method of producing the textured layer is biocompatible, such that it is innately non-toxic and does not leave a toxic residue. This is of particular importance where the structured silicone rubber is intended for use in tissue culture and medical applications, although a number of further factors also need to be considered when choosing a suitable sacrificial filler. For example, the sacrificial filler should preferably not react with the silicone rubber, either in its precursor form or in its cured state. The filler should also preferably be soluble in order to facilitate its removal by dissolution and the solvent used to dissolve the material should preferably not react with the silicone rubber. If the silicone rubber is to be cured at elevated temperatures, it is usually desirable to use a sacrificial filler that is stable at the curing temperatures, since materials that melt or decompose at high temperatures may be unsuitable, particularly if a structured silicone rubber having a high degree of regularity is desired. Finally, for commercial reasons, it is generally desirable that the sacrificial filler should be relatively inexpensive and readily available. In a preferred embodiment, the sacrificial filler is ground, prior to contacting the silicone rubber precursor. This has the advantage of allowing the resultant structure of the silicone rubber to be controlled much more accurately. Any suitable method for grinding the sacrificial filler may be used, although it has been found that wet-milling the sacrificial filler, prior to mixing with the silicone rubber precursor, gives good results. However, the sacrificial filler may also be ground by dry milling, preferably under an inert or dry atmosphere, such as under dry nitrogen or argon gas. In a preferred embodiment, the sacrificial filler is milled to a particle size of 0.005-1mm, preferably 0.01-0.5mm, and most preferably 0.05-0.1mm. These particle sizes produce micro-cupels of the same size in the resultant textured silicone layer. In a further embodiment, the sacrificial filler is granular and, preferably, crystalline, although certain amorphous fillers may also be suitable. Inorganic salts have been found to give particularly good results, although certain crystalline organic compounds, such as simple saccharides, may often be equally effective. Where the sacrificial filler is an inorganic salt, it is especially preferred to grind it first by milling it in an organic solvent, since this gives good control over resultant particle size. Preferably, the sacrificial filler is an inorganic salt selected from the group consisting of metal halides, metal carbonates and metal bicarbonates, especially one selected from the group consisting of lithium bicarbonate, sodium bicarbonate, potassium bicarbonate, lithium chloride, sodium chloride and potassium chloride. In an especially preferred embodiment, the sacrificial filler is sodium bicarbonate or sodium chloride, preferably of high purity, such as US Pharmacopoeia grade sodium bicarbonate or sodium chloride. In this last embodiment, the sodium bicarbonate or sodium chloride is preferably wet-milled under xylene, although other volatile organic solvents may also be used. In a further embodiment, the ground sacrificial filler is classified, prior to contacting the silicone rubber precursor to ensure uniform particle size distribution, for example, by passing the ground material through sieves or by using a Malvern® Particle Sizer. In another embodiment, the sacrificial filler is removed by dissolution, preferably in an aqueous solvent. In the latter case, the sacrificial filler is desirably chosen so that it does not cause swelling of the silicone rubber when removed with an aqueous solvent.

In one preferred embodiment of the present invention, the micro-cupels on the interior surface of the culture chamber are larger than those on the exterior surface. The larger micro-cupels help to promote cell adhesion inside the culture chamber.

There are several ways in which cell adhesion to the textured silicone layer may be enhanced. In one embodiment, at least a portion of the free groups that are normally present in the silicone rubber are chemically modified, for example using potassium hydroxide or sodium hydroxide, thereby converting them into -OH groups. These -OH groups are then chemically converted to form positively charged groups, for example, by reaction with diethylaminoethylbromide to give DEAE moieties, or to form negatively charged groups, for example, by reaction with iodoacetic acid, to give carboxylate moieties. Such chemical modification of groups serves to enhance or promote cell adherence. In an alternative embodiment, the surface of the silicone rubber may be charged electrostatically, for example, by bombardment with electrons. Alternatively, the surface characteristics of the silicone rubber may be modified by applying a thin coating of a suitable polymer, so as to make it more adherent to certain cells, whilst still retaining a sufficient degree of gas permeability. Any suitable bio-active polymer may be used, such as one selected from the group consisting of proteins, lipoproteins, lipopolysaccharides and polysaccharides. In a preferred embodiment, the silicone rubber precursor also includes at least one additive that is not removed with the sacrificial filler and serves to impart desired physical properties on the resultant silicone rubber. For example, the additive may be a metal powder or carbon black, which can be used to render the silicone rubber electrically conductive. Alternatively, the additive may be stainless steel powder or iron oxide, which can be used to increase the density of the silicone rubber. The additive may also be an inert substance, such as glass, which can be used to render the silicone rubber mechanically rigid. However, many other suitable additives will also be apparent to those skilled in the art.

Preferably, the culture chamber has at least one port extending between the interior and the exterior of the chamber. More often, however, there will be at least two ports, preferably including an inlet and an outlet port. In addition, one or more septum ports may also be provided, to reduce the risk of contamination when introducing various substances to the culture chamber. In an embodiment, at least one of the inlet and outlet ports are septum ports.

In a further embodiment of the invention, the surface area available for cell adhesion is further-increased inside the culture chamber by including one or more additional cell attachment means. In one embodiment, the additional cell attachment means is one or more gas-permeable sheets, which are made in the same way as the textured gas-permeable sheets described above. Preferably, these sheets are also textured on both of their surfaces, thereby providing a large surface area. In an alternative embodiment, the additional cell attachment means is a textured semi-permeable sheet. In yet another embodiment, the additional cell attachment means is a porous structure, the pores providing a surface to which the cells become anchored within the culture chamber. In a preferred embodiment, the porous additional cell attachment means is porous silicone rubber. The porous additional cell adhesion means may be in the form of sheets or pellets.

Preferably, the porous silicone is made by mixing a sacrificial filler into uncured silicone rubber, so that it becomes dispersed throughout the rubber. Next, the rubber is cured and finally the sacrificial filler is removed, leaving a system of pores and channels throughout the silicone rubber structure. The pores of the silicone rubber provide sites of attachment for cells or tissues, so that the cells or tissues may be substantially trapped within the resultant structure. The resultant mixture may be shaped prior to curing, preferably by moulding or extrusion. In a preferred embodiment, the average size of the pores formed is 1 µm-0.5 mm, preferably 10 µm to 0.2 mm, and most preferably 50 to 150 µm in diameter. Preferably, the porous silicone rubber is cut to a desired size or shape once cured. The inventors of the present invention have found that the use of certain sacrificial fillers can have an adverse effect on the resultant silicone rubber. For example, the use of sodium chloride can cause the silicone rubber to swell, depending upon the conditions. In order to avoid, this, it is desirable to use a sacrificial filler that does not cause swelling or adversely effect the resultant silicone rubber. Sodium bicarbonate has been found to be particularly effective in satisfying such criteria, although a number of other sacrificial fillers may be equally effective. If sodium bicarbonate is to be used as a sacrificial filler, it decomposes and, therefore, "blows" the material at temperatures above approximately 180°C. Consequently, it is necessary to adapt the manufacturing process so as to avoid temperatures above 180°C, for example, by selecting silicone rubbers, which cure at lower temperatures. Many of the alternative sacrificial fillers are toxic, leave toxic residues when dissolved, or are problematic at moderate temperatures required for working with silicone rubber. The additional cell attachment means may be either held in place within the culture chamber, or it may be "free-floating". Where the additional cell attachment means is a sheet, it may be used to divide the culture chamber into two or more compartments. Where the additional cell attachment means comprises a semi-permeable membrane, the contents of the two or more chambers will remain separate. The compartments within the culture chamber may either share inlet/outlet ports or have separate ones so that the contents of the compartments can be controlled independently of one another. The use of such additional cell attachment means is only worthwhile where the culture chamber provides sufficient gaseous exchange between the chamber interior and exterior to supply the increased number of cells with enough oxygen, whilst also removing the gaseous waste products.

In a second aspect of the invention, the culture chamber according to the first aspect of the present invention is in the form of a flexible bag or envelope. In recent years, flexible culture bags have become increasingly popular, offering a number of advantages over traditional cell culture apparatus, such as multi-well plates, flasks, roller bottles and spinner flasks. For example, culture bags represent closed systems, thus reducing the risk of contamination, as well as taking up less storage and incubator space. In addition such culture bags can often be produced relatively inexpensively, making them effectively disposable and reducing any need to sterilise them for re-use. Silicone rubbers have a relatively high oxygen permeability compared to most known polymers, and tubing or membranes made from such materials are well-suited for use in cell culture, where they are able to provide improved diffusion of oxygen to the cells. Silicone rubbers not only provide gas permeability (including oxygen and carbon dioxide) but also vapour transmission, structural integrity, resilience and temperature resistance, all of which are desirable in cell and tissue culture. International patent publication no. WO 97/21347 (Avecor Cardiovascular, Inc.) discloses a cell culture bag formed from a plurality of thin, spaced, gas-permeable silicone membranes, whose gas exchange rate is claimed to be significantly higher than most conventional culture bags. However, although such bags may be capable of sustaining higher cell densities and cell viability, they are ultimately limited by the surface area of the bag. Moreover, the interior surfaces of such bags are smooth and, thus, provide poor cell attachment features, making them unsuitable for efficient cell culture of anchorage-dependent cells. Furthermore, certain "problem" cell types are unable to attach to the smooth interior surface of the bags. An elaborate (and seemingly expensive) method of increasing the surface area available for cell adhesion is described in United States patent no- 4,937,194 (Baxter International, Inc.), which discloses a flexible bag containing an internal cellular structure, such as a honeycomb type structure with hexagonal channels passing through it, serving as adherent sites for cells being cultured. This document also proposes the use of microcarriers, such as small glass spheres or sodium alginate, to increase the surface area available for cell adherence inside the bag. There is a need, therefore, to overcome some of the aforementioned disadvantages.

The culture bag of the present invention provides an increased growth substrate surface area for cell attachment, as well as providing increased gaseous exchange between the bag interior and exterior. Moreover, the bag structure is simple and inexpensive to manufacture. In a preferred embodiment, the bag is made from at least one textured gas permeable membrane as described above and preferably all of the walls making up the bag comprise such membranes. As already described above in relation to the culture chamber, the culture bag preferably also includes one or more ports, extending between the bag interior and bag exterior. Such ports may be used for introducing nutrient medium, taking samples, adding further ingredients, etc. The ports should preferably have valves, locks or the like, to avoid contamination of the bag interior. In a preferred embodiment, the culture bag is provided with an inlet and an outlet port with luer locks, and a septum port for taking samples or introducing substances into the bag. The ports are desirably positioned between the sealed edges of the culture bag. It has been found that the application of a textured surface to a culture bag wall in accordance with the invention can result in the wall becoming opaque. In a preferred embodiment, therefore, the culture bag also includes at least one portion of membrane to which no textured surface layer has been applied, this area serving to act as a transparent window, thus allowing a user to see inside the culture chamber. In a further embodiment, the culture chamber also includes a valve means, allowing the release of gases that build up during cell growth and may form an air bubble inside the bag. The presence of a bubble within the chamber can prevent colonisation on the surface area adjacent the bubble because the surface will not be in contact with the culture medium. Thus, the presence of a valve in the culture chamber wall helps to minimise the size of any gas bubbles, thereby allowing a larger surface area of the bag to remain in contact with the nutrient medium and to be available for cell attachment. Almost complete colonisation on the interior chamber surface is, therefore, possible, increasing the efficiency of the culture chamber. Desirably, the valve comprises a filter means, allowing gasses to diffuse out of the chamber but preventing microbial contamination. In a preferred embodiment, the valve means comprises one or more layers of a hydrophobic material, such as a hydrophobic PTFE membrane, preferably having a thickness of around 0.25 mm and a porosity of 0.2 microns. However, other suitable forms of valve means will also be apparent to those skilled in the art. In a preferred embodiment, the culture bag further comprises a second chamber separated from the first chamber by means of a semi-permeable membrane. The second chamber preferably has an access means separate from that of the first chamber.

In a third aspect of the invention, there is provided an apparatus comprising a plurality of culture chambers according to the invention in its first aspect, for use in a method of culturing microbiological material. In an embodiment, the inlets of the culture chambers are interconnected and the outlets of the culture chambers are interconnected. In a preferred embodiment, the apparatus has at least one further chamber(s) having a semi-permeable wall that is positioned within each culture chamber, each semi-permeable chamber(s) having an inlet that is interconnected with the inlet of any other semi-permeable chambers and having an outlet that is interconnected with the outlet of any other semi-permeable chambers. In a preferred embodiment, the apparatus is filled with liquid medium, which has first been inoculated with a desired cell line. The assembly of reactor tubes may then be arranged to be rotated or agitated, for example, using machinery such as that employed for conventional roller bottles. Rotation may be continued until cell confluence is obtained, as evidenced by the levelling of the rate of glucose uptake. The inner surfaces of the reactor tubes are, therefore, extensively coated with the cells as this stage. If appropriate, rotation may be interrupted for replacement of the medium in the reactor. The reactor tubes can then be removed from the rollers and connected to a suitable media reservoir. A continuous stream of liquid nutrient medium may be arranged to pass through the reactor envelopes, the product being harvested at the outlet. During this procedure, it is desirable to provide airflow over the reactor, to assist oxygenation. The productivity and efficiency of the growth process, especially in the case of anchorage dependent cells, can be substantially enhanced using the bio-reactors according to the invention, especially when compared with conventional reaction vessels that do not utilise oxygen permeable containers and, thus, cannot sustain cell growth process in the manner permitted by the invention.

In another embodiment, the culture chambers further include semi-permeable chambers positioned within them, such as, for example, semi-permeable chambers made of cellulose acetate. The semi-permeable chambers are arranged to be separately connected to common inlets and outlets at their respective ends. In this embodiment, the bio-processing operation involves the following procedures. First, the cells grown to perform the bio-processing function are attached to the textured surface of the culture chamber using the method described above. The culture medium is then removed from said culture chambers. Next, the nutrient medium is passed through the culture chambers textured interior surface, which are now coated with cells. The media is introduced from a reservoir through the inlet at one end of the culture chambers, issuing at the outlet on the opposing end. If desired, the medium may be recycled from the outlet, to return again to the inlet of the chambers. The liquor to be processed, such as blood, for example, is then arranged to flow through the semi-permeable chambers. The liquor is introduced for this purpose at the inlet of the semi-permeable chambers, and issuing at the outlet on the opposing end. The liquor is preferably passed through the semi-permeable chambers in opposing direction to that of the nutrient medium. During this procedure, nutrients from the medium feed the cells adhering to the coating of the culture chambers. At the same time, waste materials in the process liquor, such as ammonia, pass across the semi-permeable membrane and are processed to harmless compounds by the cells in the culture chamber. In addition, advantageous products produced by the cells in the culture chamber pass back across the membrane into the process liquor. The treated liquor is finally collected at the outlet of the culture chambers. In a preferred embodiment, the apparatus is especially adapted for bio-processing of liquors containing particulate matter, such as blood cells or cell debris. The continuous flow system according to the invention is especially applicable to the processing of whole blood, for example, in an artificial *extra corporeal* organ substituting or supporting the functions of the human liver. Advantageously, the system obviates the need of separating the particulate matter prior to processing and then having to reunite the constituents. It is also envisaged that the culture chambers and apparatus according to the invention may have other medical applications, such as for expansion of other primary cell types, or for use as an *ex vivo* model for drug metabolism if colonised with hepatocytes and the like. In a yet further embodiment, the culture chamber is closed and there is a flow of media through the semi-permeable membrane that feeds the cells within the culture chamber. This means that both the cells and the cell products will become concentrated within the culture chamber, as neither is able to pass out through the membrane. This concentrated solution of cells and/or product can then be harvested periodically through ports which communicate with the interior of the culture chamber.

In a fourth aspect of the invention, the culture chamber according to the first aspect of the invention is a well for use in a method of culturing microbiological material. In a preferred embodiment, the textured gas-permeable portion of the wall is positioned at or near to the base of the well.

In a fifth aspect of the invention, there is provided a microtitre plate having at least one well according to the invention in its fourth aspect. The wells help to increase both the quantity of cells that can be grown in a microtitre well of a given size, as well as their metabolic activity. As microtitre wells become increasingly minimised in size, the number of cells that can be grown in each well, for example, for drug metabolism studies, is also reduced because of the decrease in available growth surface area. Moreover, those cells that can be grown are also starved of oxygen due to the decrease in gassing surface to volume ratio. The microtitre plate according to the invention helps to alleviate these problems by firstly increasing the gaseous exchange between the interior and exterior of the well and by increasing the available surface area for cell attachment.

In order that the invention may be better understood, examples of the various aspects will now be described, by way of illustration only and with reference to the accompanying drawings, wherein:
Figure 1 is a cross-sectional view of a membrane wall of the culture chamber in accordance with the present invention;
Figure 2 is a schematic cross-sectional view of a simple culture chamber in accordance with the invention;
Figure 3 is a schematic cross-sectional view of a culture chamber with additional textured membranes positioned within the chamber;
Figure 4 is a schematic cross-sectional view of a culture chamber used for concentrating the cells and their products;
Figure 5 is a schematic diagram of the movement of nutrients and waste, etc. within the culture chamber shown in Figure 4;
Figure 6 is a schematic cross-sectional view of a culture chamber used as a bio-reactor;
Figure 7 is a schematic diagram of the movement of nutrients and waste, etc. within the culture chamber shown in Figure 6;
Figure 8 is a schematic plan view of a culture bag in accordance with the second aspect of the invention;
Figure 9 is a schematic cross-sectional view of the culture bag of Figure 8;
Figure 10 is an exploded view of the valve of the culture bag of Figures 8 and 9;
Figure 11 is a diagrammatic illustration of a bio-reactor apparatus according to the third aspect of the invention;
Figure 12 is a silicone rubber tube from the bio-reactor of Figure 11;
Figure 13 is a cross-sectional side view of a bio-reactor apparatus with dialysis tubes;
Figure 14 shows plan view of a mictrotitre plate according to the fifth aspect of the invention;
Figure 15 shows a cross-sectional view of the plate of Figure 14 along line A-A';

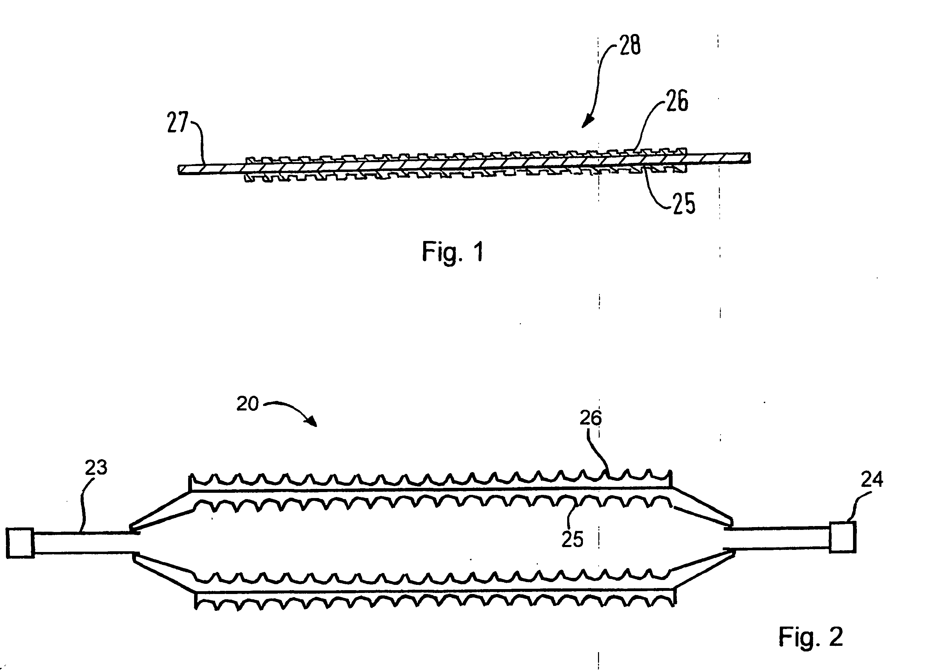

Figure 1 shows a textured membrane 28 which will form part of the wall of the culture chamber according to the first aspect of the present invention. The structure comprises a gas-permeable membrane 27, and textured surfaces 25 and 26. Each bag membrane 28 is prepared by covering the edges 27 of a smooth silicone rubber sheet with a mask and applying a layer of room-temperature vulcanising liquid silicone rubber to the exposed central portion of the sheet. Next, vacuum-dried salt is sprinkled over the layer of liquid silicone rubber so that it is uniformly covered. The liquid silicone rubber is then cured and the salt is washed out, producing a membrane 28 with a cratered or micro-cupulated surface 26. This process is carried out to produce textured layers on both surfaces of the gas-permeable membrane.

Figure 2 shows the membrane of Figure 1 incorporated into a culture chamber. The chamber comprises two textured gas-permeable membranes which are welded together along their outer edges to form a culture bag. Inlet and outlet ports 23 extend between the inside and the outside of the bag, each port 23 being provided with a lock or valve 24. Nutrient medium may be introduced into the chamber and removed from the chamber via these ports. The culture chamber may be provided with a continuous flow of nutrient medium, or it may be filled with medium and then sealed using the valves or locks for simple batch processing.

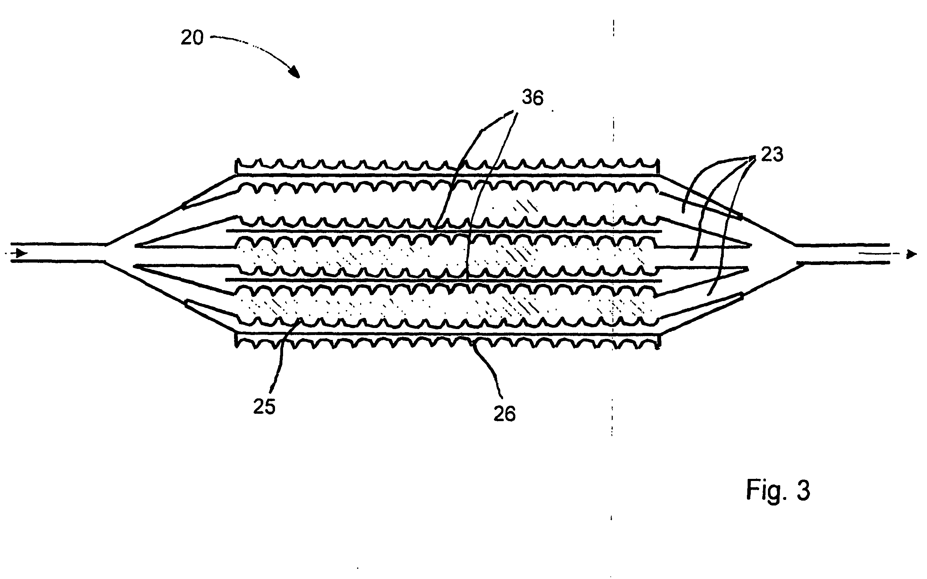

Figure 3 shows a multi-layer perfusion system. The culture chamber includes a number of additional membranes. The additional sheets 36 are textured on both surfaces. They may be sealed to one another around their edges, thereby creating separate compartments. These compartments may then be provided with medium by individual inlet/outlet ports. Alternatively, the additional sheets 36 may not be sealed along at least one edge, so that the nutrient medium can move around the chamber and the between the additional sheets.

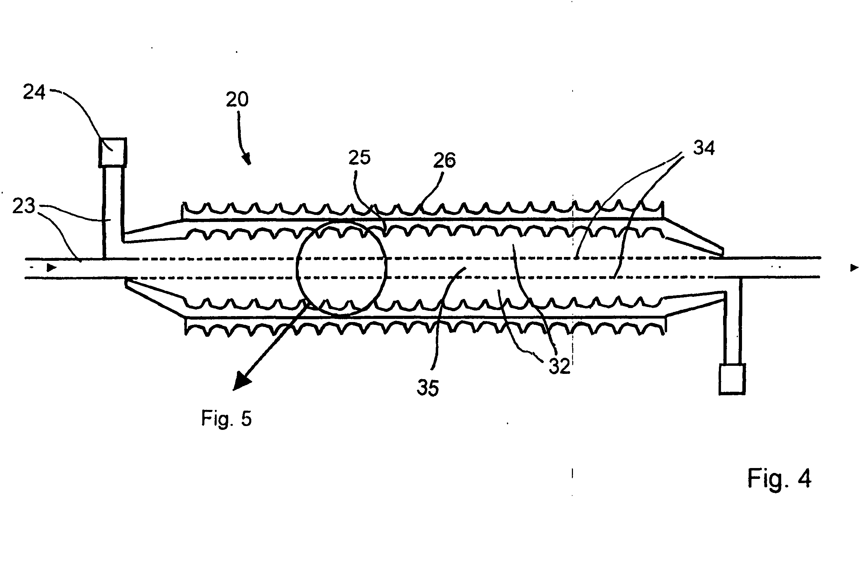

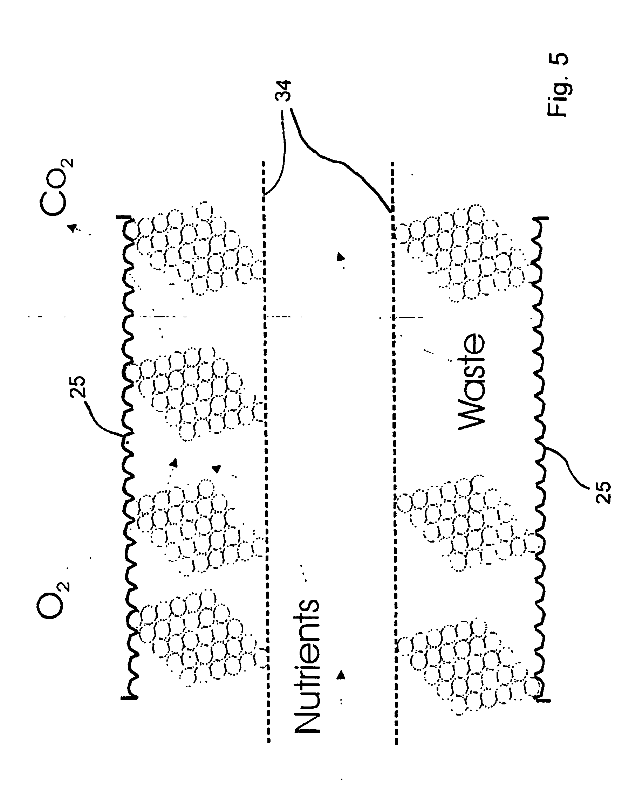

Figure 4 shows a culture chamber wherein the chamber is divided into three compartments by semi-permeable membranes 34 positioned inside the culture chamber. This culture chamber is used as a cell/cell product concentration device. The innermost compartment 35 is provided with a continual throughput of nutrient medium. The other, outermost compartments 32 contain cells. As shown in Figure 5, the nutrients from the medium diffuse through the semi-permeable membranes 34 into the outermost compartments. Oxygen diffuses into the outermost compartments 32 through the external culture chamber wall. Waste products diffuse out of the compartments containing the cells through the semi-permeable membrane 34 and flows out of the culture chamber. Carbon dioxide produced by the cells diffuses out through the external culture chamber membrane. The cells or their products may be harvested from the otherwise closed compartments 32 through the ports 23 with valves or locks 24.

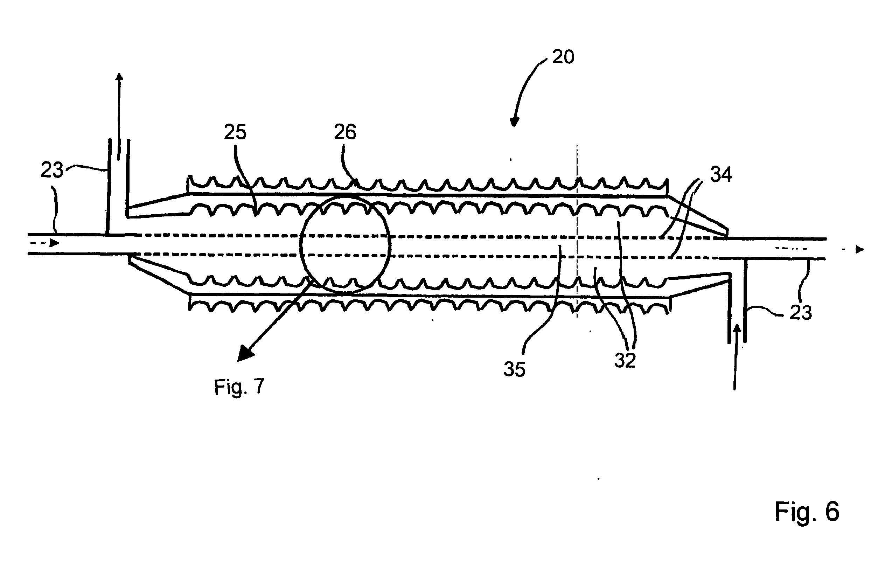

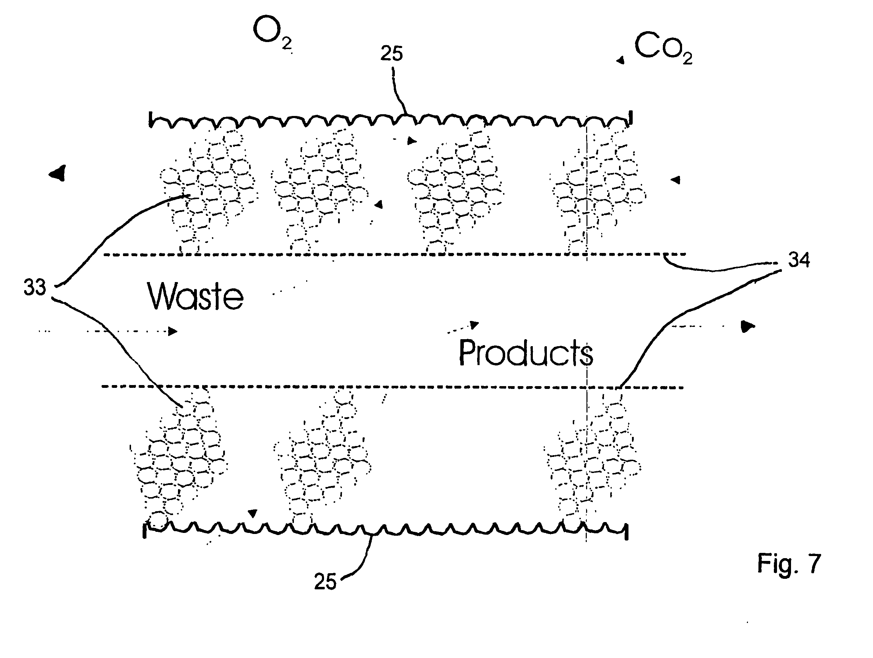

Figure 6 shows a culture chamber wherein the chamber is divided into three compartments by semi-permeable membranes 34 positioned inside the culture chamber. This culture chamber is used as a bio-reactor. The innermost compartment 35 is provided with a continual throughput of liquor which is to be processed. The other, outermost compartments 32 contain bio-processing cells, such as hepatocytes, and are provided with a throughput of fresh nutrient medium. As shown in Figure 7, the nutrients required by the cells are provided by the nutrient medium and waste products are "washed away" as the medium passes through the compartment. Oxygen diffuses into the outermost compartments 32 through the external culture chamber wall and carbon dioxide produced by the cells diffuses out through the external culture chamber membrane. The cells in the outermost compartments process waste products from the liquor in the innermost compartment 36 which diffuse through the semi-permeable membrane 34. The products of the bio-processing cells diffuse into the innermost compartment, so that processed liquor is removed from the culture chamber.

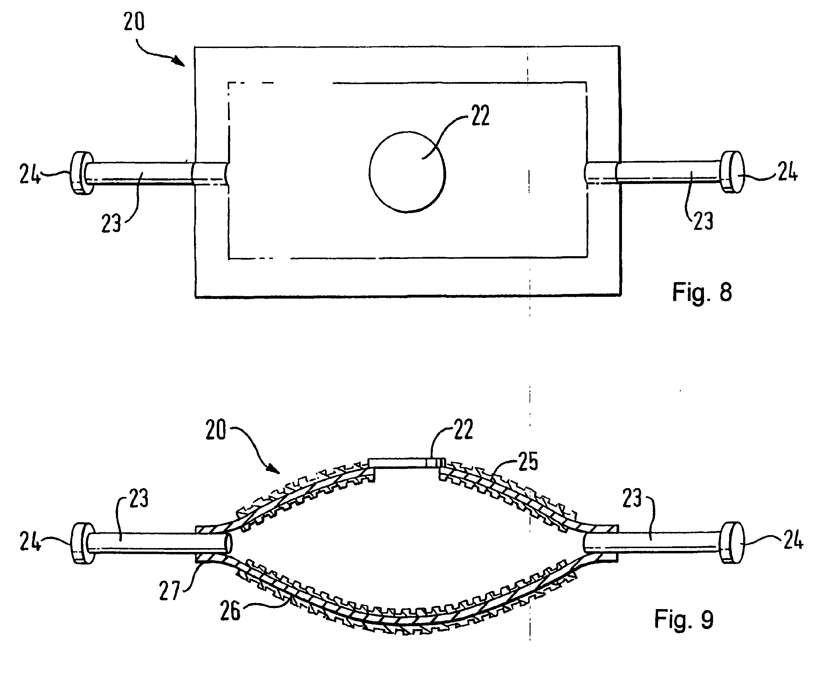

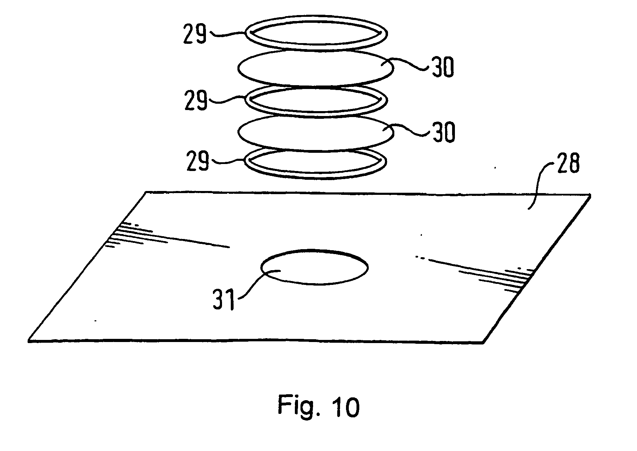

In Figures 8 and 9, a culture bag 20 comprises two membranes 28 joined at their outer edges 27, each membrane 28 having textured interior and exterior surfaces 26. A degassing valve 22 is provided in the centre of one of the membranes 28, this membrane 28 being uppermost when the bag 20 is in use. As shown in Figure 10, a degassing valve is formed by first cutting a hole 31 out of the centre of one of the membranes 28, over which the valve will be placed. A washer 29 made of uncured silicone rubber is positioned around the hole 31 on the outer face of the membrane 28. A hydrophobic PTFE membrane 30 with 0.2 µm pores and a thickness of 0.25 mm is laid over the washer 29, and a second washer 29 is placed on top. This is then repeated with a second PTFE membrane 30 and a third washer 29. When the bag 20 is to be assembled, the two silicone rubber membranes 28 are laid on top of one another. Two lengths of tubing for the inlet and outlet ports 23 are placed between the silicone rubber membranes 28, protruding slightly into what will be the interior of the assembled bag. The ports 23 are provided with valves 24. Next, room temperature vulcanising silicone rubber is applied to the untreated, smooth edges 27 of the silicone membranes 28, along which the membranes 28 are to be joined to form a bag configuration 20. Uncured silicone rubber is applied around the tubing where it lies adjacent to the smooth edges of the membranes 28. The constituents of the culture bag 20 so arranged are then welded or glued together using elevated temperatures and pressure. The edges of the silicone membranes 28 are sealed to form a bag 20, the degassing valve is formed from the layers of washers 29 and PTFE membrane 30, and the tubing for the ports 23 becomes integrated into the bag structure 20. In an alternative method, a gasket of uncured silicone rubber is placed between the membranes at their smooth edges. Upon the application of pressure and temperature, the gasket cures, fusing the edges of the membranes together to form the culture bag. The optional one or more additional cell attachment means may be appropriately positioned between the bag walls during the above described assembly method. Where the additional cell attachment means is a sheet, it may be welded to the bag walls along its edges, thereby creating compartments within the culture bag.

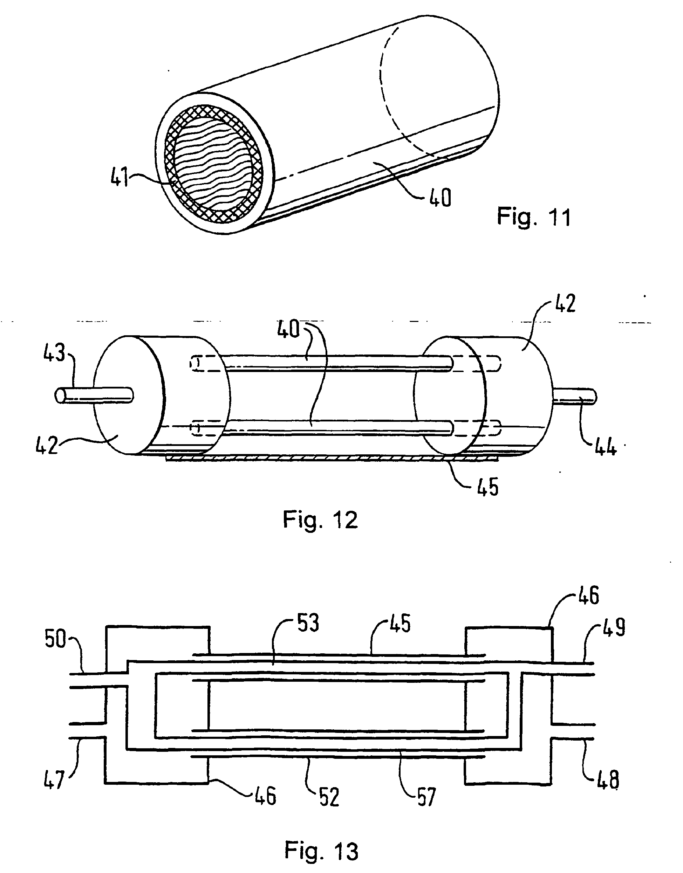

In Figure 11, a bio-reactor apparatus comprises two reactor tubes 40 (in practice, a larger number, such as seven or eight tubes, is generally preferred). Each reactor tube 40 carries internal and external coatings of textured silicone rubber 41. In use, in order to grow the cells on interior surfaces 41 of tubes 40, medium carrying cell lines is introduced through an inlet 43. Reactor tubes 40 are interconnected through distributors 42. One or more strengthening members 45 ensure rigidity of the assembly. The assembly is rotated on rollers (not shown), followed by evacuation of the liquid and subsequent passage of nutrient medium over the cells. The medium is introduced through the inlet 43 and issues from the outlet 44. The product is finally collected at the outlet 44. In Figure 12, the reactor comprises a non-porous silicone rubber tube 40 carrying internal and external coatings of textured silicone rubber 41. In Figure 13, dialysis tubes 51 are co-axially positioned within the reactor tubes 40. Cells are grown in the annular space 52 by the passage *via* introduction of medium comprising the cell line through the inlet 47. After removal of the liquid from the annular space through the outlet 48, the medium to be processed is passed through the dialysis tubes 51 via medium inlet 49, issuing at outlet 50. At the same time, the liquor to undergo the bio-reaction is passed through the reactor tubes via inlet 47, for collection at outlet 48.

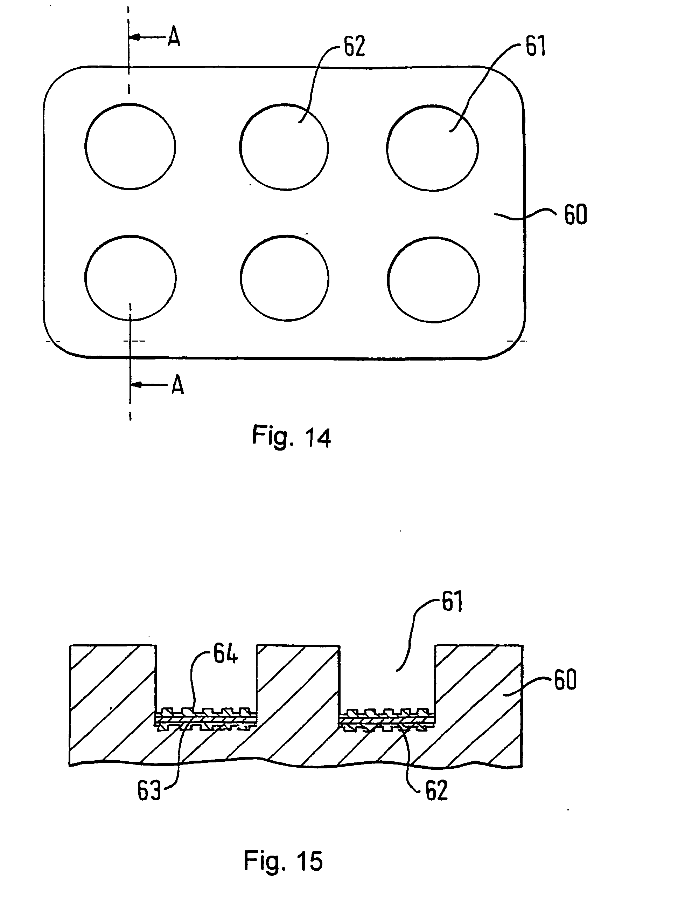

In Figures 14 and 15, a standard microtitre plate 60 has wells 61 without base walls (either conventional microtitre plates are used and the base walls of the wells removed, or a microtitre plate is produced without any base walls). A non-porous silicone membrane 62 is attached to the bottom of the wells, the membrane comprises a silicone rubber sheet 63 having a textured surface 64 on both of its sides.

## Claims

1. A culture chamber for use in a method of culturing microbiological material, which comprises a wall or at least a portion of a wall consisting of a gas-permeable membrane, a textured interior growth surface arranged for contact with the microbiological material being cultured and at least a portion of the external surface gas-permeable membrane being textured to enhance gas permeability of the membrane, and wherein the textured interior growth surface is formed on the interior surface of the gas-permeable membrane.

2. A culture chamber as claimed in claim 1, wherein the textured portion on the interior surface does not overlap with the textured portion on the exterior surface of the gas-permeable membrane.

3. A culture chamber as claimed in claim 1, wherein at least a portion of the textured interior surface and a portion of the textured exterior surface of the gas-permeable membrane overlap.

4. A culture chamber as claimed in claim 3, wherein at least 25% of the gas-permeable membrane is textured on both its interior and exterior surfaces, preferably at least 50% of the gas-permeable membrane is textured on both its interior and exterior surfaces, and most preferably at least 90% of the gas-permeable membrane is textured on both its interior and exterior surfaces.

5. A culture chamber as claimed in any of the preceding claims, wherein the gas-permeable membrane and the textured surfaces are each formed from an organic polymer.

6. A culture chamber as claimed in any of the preceding claims, wherein the gas-permeable membrane and the textured surfaces are each formed of the same organic polymer.

7. A culture chamber as claimed in any of the preceding claims, wherein at least the gas-permeable membrane consists of a silicone rubber.

8. A culture chamber as claimed in any one of the preceding claims, wherein at least one of the textured surfaces is formed by applying a layer of uncured silicone to the gas permeable membrane, applying a sacrificial filler to the uncured layer, curing the silicone rubber, and removing the sacrificial filler.

9. A culture chamber as claimed in claim 8, wherein the sacrificial filler particles have a diameter of 0.005-1mm, preferably 0.01-0.5mm, and most preferably 0.05-0.1mm.

10. A culture chamber as claimed in any of the preceding claims, wherein the textured surfaces of the gas-permeable membrane are micro-cupellated.

11. A culture chamber as claimed in claim 10, wherein the size of the micro-cupels on the interior surface of the culture chamber are different to the size of those on the exterior surface of the culture chamber.

12. A culture chamber as claimed in claim 11, wherein the micro-cupels on the interior surface of the culture chamber are larger than those on the exterior surface.

13. A culture chamber as claimed in any one of claims 10-12, wherein the micro-cupels have a diameter of 0.005-1mm, preferably 0.01-0.5mm, and most preferably 0.05-0.1mm.

14. A culture chamber as claimed in claim 8, wherein a thicker layer of uncured silicone is applied, thereby allowing the particles of sacrificial filler to sink into said layer and become overlaid with further particles of sacrificial filler, and to form deep micro-cupels and channels in the cured silicone layer.

15. A culture chamber as claimed in claim 14, wherein the thicker layer of silicone rubber is on the interior surface of the culture chamber.

16. A culture chamber as claimed in claim 15, wherein the thicker layer on the interior surface is at least double the thickness of the layer on the exterior surface.

17. A culture chamber as claimed in any one of claims 10-16, wherein the number of micro-cupels per unit area is greater on the interior surface than on the exterior surface.

18. A culture chamber as claimed in any one of claims 10-16, wherein the number of micro-cupels per unit area is greater on the exterior surface than on the interior surface.

19. A culture chamber as claimed in any one of the preceding claims, including at least one access port extending between the interior and the exterior of the chamber.

20. A culture chamber as claimed in claim 19, including an inlet port and an outlet port.

21. A culture chamber as claimed in claims 19 or 20, including at least one septum port.

22. A culture chamber as claimed in any one of the preceding claims, wherein the interior textured surface of the gas-permeable membrane is modified to increase cell adherence.

23. A culture chamber as claimed in claim 22, wherein the interior textured surface of the gas-permeable membrane is charged by bombardment with electrons.

24. A culture chamber as claimed in claim 22, wherein the free groups of the silicone rubber are converted to -OH groups, the -OH groups then being modified by the attachment of chemical moieties.

25. A culture chamber as claimed in any one of the preceding claims, containing an additional cell attachment means within the culture chamber.

26. A culture chamber as claimed in claim 25, wherein the means for increasing the surface area for cell attachment comprises a further membrane, preferably with at least one textured surface.

27. A culture chamber as claimed in claim 26, wherein the membrane is gas-permeable or porous.

28. A culture chamber as claimed in claim 26, wherein the membrane is semi-permeable and divides the culture chamber into two separate chambers.

29. A culture chamber as claimed in claim 28, wherein the two separate chambers have separate access ports.

30. A culture chamber as claimed in any one of the preceding claims, wherein the culture chamber is in the form of a flexible bag or envelope.

31. A culture chamber as claimed in claim 30, wherein the flexible bag or envelope is made of silicone rubber.

32. A culture chamber as claimed in any one of claims 30 or 31, including a valve means for release of gasses that build up within the chamber during use.

33. A culture chamber as claimed in claim 32, wherein the valve means comprises at least one filter means, the filter means allowing gasses to diffuse out of the chamber, but preventing microbial contamination thereof.

34. A culture chamber as claimed in claims 32 or 33, wherein the valve means comprises one or more layers of a hydrophobic porous material.

35. A culture chamber as claimed in claim 34, wherein the hydrophobic membrane is a PTFE membrane having a thickness of 0.25 mm and a porosity of 0.2 µm.

36. An apparatus comprising a plurality of culture chambers as claimed in any of the preceding claims, for use in a method of culturing microbiological material.

37. An apparatus as claimed in claim 36, wherein the inlets of the culture chambers are interconnected and the outlets of the culture chambers are interconnected.

38. An apparatus as claimed in claims 36 or 37, wherein a further chamber having a semi-permeable wall is positioned within each culture chamber, each semi-permeable chamber having an inlet that is interconnected with the inlet of the other semi-permeable chambers and having an outlet that is interconnected with the outlet of the other semi-permeable chambers.

39. A culture chamber as claimed in any of claims 1-28, wherein the chamber is a well.

40. A well as claimed in claim 39, wherein the textured gas-permeable membrane is positioned at the base of the well.

41. A microtitre plate having at least one well as claimed in claims 39 or 40.

## Patentansprüche

1. Kulturkammer zur Verwendung in einem Verfahren zur Kultivierung mikrobiologischen Materials, umfassend eine Wand oder mindestens einen Bereich einer Wand, bestehend aus einer gaspermeablen Membran, eine strukturierte innere Wachstumsoberfläche, die für den Kontakt mit dem kultivierten mikrobiologischen Material eingerichtet ist, und mindestens einen Bereich der äußeren Oberfläche der gaspermeablen Membran, der zur Verbesserung der Gaspermeabilität der Membran strukturiert ist, und wobei die strukturierte innere Wachstumsoberfläche auf der inneren Oberfläche der gaspermeablen Membran ausgebildet ist.

2. Kulturkammer nach Anspruch 1, wobei der strukturierte Bereich auf der inneren Oberfläche nicht mit dem strukturierten Bereich auf der äußeren Oberfläche der gaspermeablen Membran überlappt.

3. Kulturkammer nach Anspruch 1, wobei mindestens ein Bereich der strukturierten inneren Oberfläche und ein Bereich der strukturierten äußeren Oberfläche der gaspermeablen Membran überlappen.

4. Kulturkammer nach Anspruch 3, wobei mindestens 25% der gaspermeablen Membran sowohl auf deren innerer als auch deren äußerer Oberfläche strukturiert sind, vorzugsweise mindestens 50% der gaspermeablen Membran sowohl auf deren innerer als auch deren äußerer Oberfläche strukturiert sind, und am meisten bevorzugt mindestens 90% der gaspermeablen Membran sowohl auf deren innerer als auch deren äußerer Oberfläche strukturiert sind.

5. Kulturkammer nach einem der vorhergehenden Ansprüche, wobei die gaspermeable Membran und die strukturierten Oberflächen jeweils aus einem organischen Polymer gebildet sind.

6. Kulturkammer nach einem der vorhergehenden Ansprüche, wobei die gaspermeable Membran und die strukturierten Oberflächen jeweils aus demselben organischen Polymer gebildet sind.

7. Kulturkammer nach einem der vorhergehenden Ansprüche, wobei zumindest die gaspermeable Membran aus einem Silikonkautschuk besteht.

8. Kulturkammer nach einem der vorhergehenden Ansprüche, wobei mindestens eine der strukturierten Oberflächen durch Aufbringen einer Schicht von ungehärtetem Silikon auf die gaspermeable Membran, Aufbringen eines Opferfüllmittels auf die ungehärtete Schicht, Aushärten des Silikonkautschuks und Entfernen des Opferfüllmittels gebildet ist.

9. Kulturkammer nach Anspruch 8, wobei die Opferfüllmittelpartikel einen Durchmesser von 0,005-1 mm, vorzugsweise 0,01-0,5 mm und am meisten bevorzugt 0,05-0,1 mm aufweisen.

10. Kulturkammer nach einem der vorhergehenden Ansprüche, wobei die strukturierten Oberflächen der gaspermeablen Membran mikrokupelliert sind.

11. Kulturkammer nach Anspruch 10, wobei sich die Größe der Mikrokupel auf der inneren Oberfläche der Kulturkammer von der Größe derjenigen auf der äußeren Oberfläche der Kulturkammer unterscheidet.

12. Kulturkammer nach Anspruch 11, wobei die Mikrokupel auf der inneren Oberfläche der Kulturkammer größer sind als diejenigen auf der äußeren Oberfläche.

13. Kulturkammer nach einem der Ansprüche 10-12, wobei die Mikrokupel einen Durchmesser von 0,005-1 mm, vorzugsweise 0,01-0,5 mm und am meisten bevorzugt 0,05-0,1 mm aufweisen.

14. Kulturkammer nach Anspruch 8, wobei eine dickere Schicht von ungehärtetem Silikon aufgebracht wird, wodurch es den Partikeln des Opferfüllmittels ermöglicht wird, in die Schicht einzusinken und mit weiteren Partikeln des Opferfüllmittels überlagert zu werden und tiefe Mikrokupel und Kanäle in der gehärteten Silikonschicht zu bilden.

15. Kulturkammer nach Anspruch 14, wobei sich die dickere Schicht aus Silikonkautschuk auf der inneren Oberfläche der Kulturkammer befindet.

16. Kulturkammer nach Anspruch 15, wobei die dickere Schicht auf der inneren Oberfläche mindestens doppelt so dick ist wie die Schicht auf der äußeren Oberfläche.

17. Kulturkammer nach einem der Ansprüche 10-16, wobei die Zahl der Mikrokupel pro Flächeneinheit auf der inneren Oberfläche größer ist als auf der äußeren Oberfläche.

18. Kulturkammer nach einem der Ansprüche 10-16, wobei die Zahl der Mikrokupel pro Flächeneinheit auf der äußeren Oberfläche größer ist als auf der inneren Oberfläche.

19. Kulturkammer nach einem der vorhergehenden Ansprüche, beinhaltend mindestens einen Zugangsanschluss, der sich zwischen dem Inneren und dem Äußeren der Kammer erstreckt.

20. Kulturkammer nach Anspruch 19, beinhaltend einen Eingangsanschluss und einen Ausgangsanschluss.

21. Kulturkammer nach Anspruch 19 oder 20, beinhaltend mindestens einen Septumanschluss.

22. Kulturkammer nach einem der vorhergehenden Ansprüche, wobei die innere strukturierte Oberfläche der gaspermeablen Membran modifiziert ist, um die Zellhaftung zu erhöhen.

23. Kulturkammer nach Anspruch 22, wobei die innere strukturierte Oberfläche der gaspermeablen Membran durch Beschuss mit Elektronen geladen ist.

24. Kulturkammer nach Anspruch 22, wobei die freien Gruppen des Silikonkautschuks zu -OH-Gruppen umgewandelt werden, wobei die -OH-Gruppen dann durch Anheftung chemischer Reste modifiziert werden.

25. Kulturkammer nach einem der vorhergehenden Ansprüche, enthaltend ein zusätzliches Zellanheftungsmittel innerhalb der Kulturkammer.

26. Kulturkammer nach Anspruch 25, wobei das Mittel zur Erhöhung der Oberfläche zur Zellanheftung eine weitere Membran umfasst, vorzugsweise mit mindestens einer strukturierten Oberfläche.

27. Kulturkammer nach Anspruch 26, wobei die Membran gaspermeabel oder porös ist.

28. Kulturkammer nach Anspruch 26, wobei die Membran semipermeabel ist und die Kulturkammer in zwei getrennte Kammern trennt.

29. Kulturkammer nach Anspruch 28, wobei die zwei getrennten Kammern getrennte Zugangsanschlüsse aufweisen.

30. Kulturkammer nach einem der vorhergehenden Ansprüche, wobei die Kulturkammer in Form eines/einer flexiblen Beutels oder Hülle vorliegt.

31. Kulturkammer nach Anspruch 30, wobei der/die flexible Beutel oder Hülle aus Silikonkautschuk hergestellt ist.

32. Kulturkammer nach einem der Ansprüche 30 oder 31, beinhaltend ein Ventilmittel zum Ablassen von Gasen, die beim Gebrauch innerhalb der Kammer entstehen.

33. Kulturkammer nach Anspruch 32, wobei das Ventilmittel mindestens ein Filtermittel umfasst, wobei es das Filtermittel Gasen ermöglicht, aus der Kammer zu diffundieren, die mikrobielle Kontamination derselben jedoch verhindert.

34. Kulturkammer nach Anspruch 32 unter 33, wobei das Ventilmittel ein oder mehrere Schichten eines hydrophoben porösen Materials umfasst.

35. Kulturkammer nach Anspruch 34, wobei die hydrophobe Membran eine PTFE-Membran mit einer Dicke von 0,25 mm und einer Porosität von 0,2 µm ist.

36. Vorrichtung, umfassend eine Mehrzahl von Kulturkammern nach einem der vorhergehenden Ansprüche, zur Verwendung in einem Verfahren zur Kultivierung mikrobiologischen Materials.

37. Vorrichtung nach Anspruch 36, wobei die Eingänge der Kulturkammer miteinander verbunden sind und die Ausgänge der Kulturkammern miteinander verbunden sind.

38. Vorrichtung nach Anspruch 36 oder 37, wobei eine weitere Kammer mit einer semipermeablen Wand innerhalb jeder Kulturkammer angeordnet ist, wobei jede semipermeable Kammer einen Eingang aufweist, der mit dem Eingang der anderen semipermeablen Kammern verbunden ist, und einen Ausgang aufweist, der mit dem Ausgang der anderen semipermeablen Kammern verbunden ist.

39. Kulturkammer nach einem der Ansprüche 1-29, wobei die Kammer ein Napf ist.

40. Napf nach Anspruch 39, wobei die strukturierte gaspermeable Membran am Boden des Napfes angeordnet ist.

41. Mikrotiterplatte mit mindestens einem Napf nach Anspruch 39 oder 40.

## Revendications

1. Enceinte de culture destinée à une utilisation dans un procédé de culture d'un matériau microbiologique, qui comprend une paroi ou au moins une partie d'une paroi constituée d'une membrane perméable aux gaz, une surface de croissance intérieure texturée disposée pour être en contact avec le matériau microbiologique qui est cultivé et au moins une partie de la surface extérieure de la membrane perméable aux gaz qui est texturée pour augmenter la perméabilité au gaz, et dans laquelle la surface de croissance intérieure texturée est formée sur la surface intérieure de la membrane perméable aux gaz.

2. Enceinte de culture selon la revendication 1, dans laquelle la partie texturée sur la surface intérieure ne recouvre pas la partie texturée sur la surface extérieure de la membrane perméable aux gaz.

3. Enceinte de culture selon la revendication 1, dans laquelle au moins une partie de la surface intérieure texturée et une partie de la surface extérieure texturée de la membrane perméable aux gaz se recouvrent.

4. Enceinte de culture selon la revendication 3, dans laquelle au moins 25 % de la membrane perméable aux gaz est texturée à la fois sur ses surfaces intérieure et extérieure, de préférence au moins 50 % de la membrane perméable aux gaz est texturée à la fois sur ses surfaces intérieure et extérieure, et de manière préférée entre toutes au moins 90 % de la membrane perméable aux gaz est texturée sur ses surfaces intérieure et extérieure.

5. Enceinte de culture selon l'une quelconque des revendications précédentes, dans laquelle la membrane perméable aux gaz et les surfaces texturées sont chacune formées à partir d'un polymère organique.

6. Enceinte de culture selon l'une quelconque des revendications précédentes, dans laquelle la membrane perméable aux gaz et les surfaces texturées sont chacune constituées du même polymère organique.

7. Enceinte de culture selon l'une quelconque des revendications précédentes, dans laquelle au moins la membrane perméable aux gaz est constituée d'un caoutchouc de silicone.

8. Enceinte de culture selon l'une quelconque des revendications précédentes, dans laquelle au moins une des surfaces texturées est formée en appliquant une couche de silicone non durci sur la membrane perméable aux gaz, en appliquant une charge sacrificielle sur la couche non durcie, en durcissant le caoutchouc de silicone, et en retirant la charge sacrificielle.

9. Enceinte de culture selon la revendication 8, dans laquelle les particules de la charge sacrificielle ont un diamètre allant de 0,005 à 1 mm, de préférence de 0,01 à 0,5 mm, et de manière préférée entre toutes de 0,05 à 0,1 mm.

10. Enceinte de culture selon l'une quelconque des revendications précédentes, dans laquelle les surfaces texturées de la membrane perméable aux gaz ont des microcoupelles.

11. Enceinte de culture selon la revendication 10, dans laquelle la taille des microcoupelles sur la surface intérieure de l'enceinte de culture est différente de la taille de celles sur la surface extérieure de l'enceinte de culture.

12. Enceinte de culture selon la revendication 11, dans laquelle les microcoupelles sur la surface intérieure de l'enceinte de culture sont plus grandes que celles sur la surface extérieure.

13. Enceinte de culture selon l'une quelconque des revendications 10 à 12, dans laquelle les microcoupelles ont un diamètre allant de 0,005 à 1 mm, de préférence de 0,01 à 0,5 mm, et de manière préférée entre toutes de 0,05 à 0,1 mm.

14. Enceinte de culture selon la revendication 8, dans laquelle une couche plus épaisse de silicone non durcie est appliquée, permettant de ce fait aux particules de la charge sacrificielle de descendre dans ladite couche et d'être recouvertes par d'autres particules de la charge sacrificielle, et de former des microcoupelles et des canaux profonds dans la couche de silicone durci.

15. Enceinte de culture selon la revendication 14, dans laquelle la couche plus épaisse de caoutchouc de silicone est sur la surface intérieure de l'enceinte de culture.

16. Enceinte de culture selon la revendication 15, dans laquelle la couche plus épaisse sur la surface intérieure est au moins le double de l'épaisseur de la couche sur la surface extérieure.

17. Enceinte de culture selon l'une quelconque des revendications 10 à 16, dans laquelle le nombre de microcoupelles par unité de surface est plus grand sur la surface intérieure que sur la surface extérieure.

18. Enceinte de culture selon l'une quelconque des revendications 10 à 16, dans laquelle le nombre de microcoupelles par unité de surface est plus grand sur la surface extérieure que sur la surface intérieure.

19. Enceinte de culture selon l'une quelconque des revendications précédentes, comprenant au moins un orifice d'accès se prolongeant entre l'intérieur et l'extérieur de l'enceinte.

20. Enceinte de culture selon la revendication 19, comprenant un orifice d'entrée et un orifice de sortie.

21. Enceinte de culture selon les revendications 19 ou 20, comprenant au moins un orifice équipé d'un septum.

22. Enceinte de culture selon l'une quelconque des revendications précédentes, dans laquelle la surface texturée intérieure de la membrane perméable aux gaz est modifiée de façon à augmenter l'adhérence des cellules.

23. Enceinte de culture selon la revendication 22, dans laquelle la surface texturée intérieure de la membrane perméable aux gaz est chargée par bombardement d'électrons.

24. Enceinte de culture selon la revendication 22, dans laquelle les groupes libres du caoutchouc de silicone sont convertis en groupes -OH, les groupes -OH étant alors modifiés par l'attachement de fragments chimiques.

25. Enceinte de culture selon l'une quelconque des revendications précédentes, contenant des moyens pour attacher des cellules supplémentaires dans l'enceinte de culture.

26. Enceinte de culture selon la revendication 25, dans laquelle les moyens pour augmenter la superficie destinée à l'attachement de cellules comprend une autre membrane, de préférence avec au moins une surface texturée.

27. Enceinte de culture selon la revendication 26, dans laquelle la membrane est perméable aux gaz ou poreuse.

28. Enceinte de culture selon la revendication 26, dans laquelle la membrane est semi-perméable et divise l'enceinte de culture en deux enceintes séparées.

29. Enceinte de culture selon la revendication 28, dans laquelle les deux enceintes séparées ont des orifices d'accès séparés.

30. Enceinte de culture selon l'une quelconque des revendications précédentes, dans laquelle l'enceinte de culture est sous la forme d'un sac ou d'une enveloppe flexible.

31. Enceinte de culture selon la revendication 30, dans laquelle le sac ou l'enveloppe flexible est fait de caoutchouc de silicone.

32. Enceinte de culture selon l'une quelconque des revendications 30 ou 31, comprenant un moyen de soupape destiné à la libération des gaz qui s'accumulent dans la chambre au cours de l'utilisation.

33. Enceinte de culture selon la revendication 32, dans laquelle le moyen de soupape comprend au moins un moyen de filtrage, le moyen de filtrage permettant aux gaz de se répandre hors de l'enceinte, mais empêchant une contamination microbienne de celle-ci.

34. Enceinte de culture selon les revendications 32 ou 33, dans laquelle le moyen de soupape comprend une ou plusieurs couches d'un matériau poreux hydrophobe.

35. Enceinte de culture selon la revendication 34, dans laquelle la membrane hydrophobe est une membrane en PTFE ayant une épaisseur de 0,25 mm et une porosité de 0,2 µm.

36. Appareil comprenant une pluralité d'enceintes de culture selon l'une quelconque des revendications précédentes, destiné à une utilisation dans un procédé de culture de matériau microbiologique.

37. Appareil selon la revendication 36, dans lequel les entrées des enceintes de culture sont interconnectées et les sorties des enceintes de culture sont interconnectées.

38. Appareil selon les revendications 36 ou 37, dans lequel une autre enceinte ayant une paroi semi-perméable est placée dans chaque enceinte de culture, chaque enceinte semi-perméable ayant une entrée qui est interconnectée avec l'entrée des autres enceintes semi-perméables et ayant une sortie qui est interconnectée avec la sortie des autres enceintes semi-perméables.

39. Enceinte de culture selon l'une quelconque des revendications 1 à 29, dans laquelle l'enceinte est un puits.

40. Puits selon la revendication 39, dans lequel la membrane texturée perméable aux gaz est placée à la base du puits.

41. Plaque de microtitrage ayant au moins un puits selon les revendications 39 ou 40.
